# EUROPEAN PATENT APPLICATION

(11) **EP 3 081 740 A1**
(43) Date of publication of application: **19.10.2016**
(21) Application number: 15290096.5
(22) Date of filing: 13.04.2015
(51) Int. Cl.: E21B 49/00, E21B 49/08, G01N 1/20, G01N 33/28

(54) **SYSTEM AND METHOD FOR SAMPLING A DRILLING FLUID EXITING A WELLBORE**

(71) Applicant: Geoservices Equipements, 95971 Roissy en France (FR)
(72) Inventor: Leducq, Paul, 67049 Paris Nord II (FR); Coste, Clement, 95971 Roissy en France (FR)
(74) Representative: Leonori, Céline

(57) **Abstract**

A system for sampling a drilling fluid exiting the wellbore in a portion of a flowline (25) having a substantially closed section, comprising :
- a first portion (80) connected in the closed-section portion of the flowline, comprising a closing device (88) movable between an open position in which the first portion defines a fluid passage between the outside and the closed-section portion of the flowline and a closed position in which the device closes the fluid passage,
- a second portion (90) configured to be inserted in the first portion , with the closing device in open position, defining a fluid passage between the outside and the closed-section portion of the flowline;
- a retaining device (106, 108) for fixing the second portion relative to the first portion,
wherein the second portion and the retaining device are configured so as to close the fluid passage defined by the first portion.

## Description

### BACKGROUND

The disclosure relates to a sampling system for sampling a drilling fluid exiting a wellbore, in a closed section portion of a flowline. A closed section portion of the flowline is a section in which the fluid is essentially surrounded by the flowline.

Some systems already exist to sample gas from a drilling fluid circulating in a closed section flowline. A previous system has been created by Geoservices. In this previous system, a first pipe is welded on the closed section portion of the flowline, an opening being formed in the flowline so that the first pipe is in fluid communication with the flowline. A second pipe is inserted in the first pipe so that a collection chamber fixed to the second pipe is situated in the drilling fluid.

### SUMMARY

The disclosure relates to a system for sampling a drilling fluid exiting the wellbore in a portion of a flowline having a substantially closed section, comprising a first portion connected in the closed-section portion of the flowline, comprising a closing device movable between an open position in which the first portion defines a fluid passage between the outside and the closed-section portion of the flowline and a closed position in which the device closes the fluid passage. The sampling system also comprises a second portion inserted in the first portion, with the closing device in open position, defining a fluid passage between the outside and the closed-section portion of the flowline and a retaining device for fixing the second portion relative to the first portion. The second portion and the retaining device are configured, when positioned on the first portion so as to close the fluid passage defined by the first portion., thereby sealing the first portion from the outside.

The disclosure also relates to an installation for analyzing gas extracted from a drilling fluid exiting a wellbore comprising a flowline for removing drilling fluid from the wellbore, having a closed section portion, a system for sampling the drilling fluid in the closed section portion of the flowline according to the disclosure, a system for extracting at least a gas from the sampled drilling fluid, connected to the sampling system and a system for analyzing the gas extracted from the drilling fluid, connected to the extraction system.

The disclosure relates as well to a method for sampling a drilling fluid exiting a wellbore in a closed section flowline with a system according to the disclosure, comprising opening the closing device, inserting the second portion in the first portion until part of the second portion is situated in the closed section flowline, fixing the second portion relative to the first portion and connecting the second portion to a suction device to extract drilling fluid from the closed section flowline.

The first portion of the sampling system may be sealed when the second portion is in place (gas flowing to the outside via the second portion) and also when it is removed from the first portion (gas being confined in the flowline), for instance for maintenance operation or when several configuration of the wellbore do not enable extraction of the gas contained in the drilling fluid.

With such a system, installation and method, gas may be sampled as close as possible from the exit of the wellbore without affecting the flowline certification, in particular concerning pressure. The passage from the sampling configuration to the maintenance configuration is quick and easy.

### BRIEF DESCRIPTION OF THE DRAWINGS

Various aspects of this disclosure may be better understood upon reading the following detailed description and upon reference to the drawings in which:
FIG. 1 is a drawing representing an installation according to an embodiment of the disclosure,
FIG.2 is perspective view of a sampling system according to an embodiment of the disclosure in a sampling configuration
FIG.3 is a perspective view of the sampling system according to FIG.2 during the insertion of a second portion in a first portion of the sampling system
FIG. 4 is an exploded view of a part of the sampling system according to FIG.2
FIG. 5 is a sectional view of a lower part of the sampling system according to FIG.2;
FIG. 6 is a perspective view of an attaching part of the sampling system according to FIG.2, in a first position,
FIG. 7 is a perspective view of an attaching part of the sampling system according to FIG.2, in a second position.

### DETAILED DESCRIPTION

One or more specific embodiments of the present disclosure will be described below. These described embodiments are examples of the presently disclosed techniques. Additionally, in an effort to provide a concise description of these embodiments, some features of an actual implementation may not be described in the specification. It should be appreciated that in the development of any such actual implementation, as in any engineering or design project, numerous implementation-specific decisions may be made to achieve the developers' specific goals, such as compliance with system-related and business-related constraints, which may vary from one implementation to another. Moreover, it should be appreciated that such a development effort might be complex and time consuming, but would still be a routine undertaking of design, fabrication, and manufacture for those of ordinary skill having the benefit of this disclosure.

When introducing elements of various embodiments of the present disclosure, the articles "a," "an," and "the" are intended to mean that there are one or more of the elements. The terms "comprising," "including," and "having" are intended to be inclusive and mean that there may be additional elements other than the listed elements. Additionally, it should be understood that references to "one embodiment" or "an embodiment" of the present disclosure are not intended to be interpreted as excluding the existence of additional embodiments that also incorporate the recited features.

In all of the following, the terms "upstream" and "downstream" are used in reference to the normal circulation direction of a fluid in a duct.

The extraction devices according to the invention are intended to be used for example in a drilling installation of a fluid production well, in particular hydrocarbons, such as an oil well.

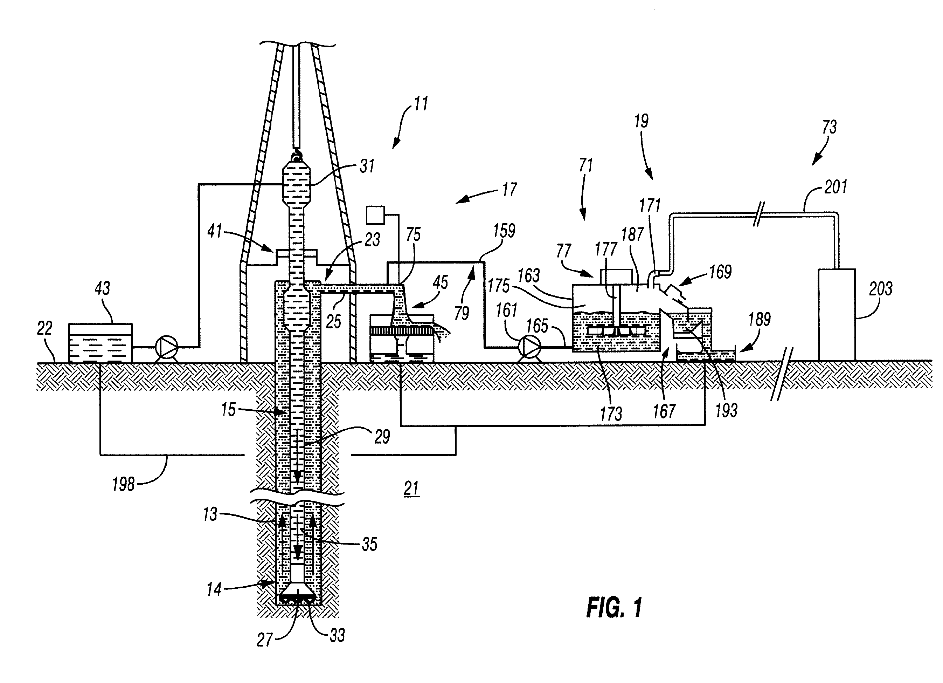

As illustrated in Figure 1, this installation 11 comprises a drilling duct 13 arranged in a cavity 14 pierced with a rotary drilling tool 15, a surface installation 17, and an analysis system 19 for the gases contained in the drilling fluid.

The drilling duct 13 is arranged in the cavity 14 pierced in the subsoil 21 by the rotary drilling tool 15. It extends in an upper portion of the height of the cavity 14 it defines. The cavity 14 also has a lower portion directly defined by the subsoil.

The drilling duct 13 includes, at the surface 22, a wellhead 23 provided with a fluid circulation duct 25.

The drilling tool 15 comprises a drilling head 27, a drill string 29, and a head 31 for injecting drilling fluid.

The drilling head 27 comprises a drill bit 33 for piercing rocks in the subsoil 21. It is mounted on the lower portion of the drill string 29 and is positioned in the bottom of the cavity 14.

The string 29 comprises a set of hollow drilling tubes. These tubes define an inner space 35 that makes it possible to bring the drilling fluid injected by the head 31 from the surface 22 to the drilling head 27. To that end, the injection head 31 is screwed onto the upper portion of the string 29.

This drilling fluid, commonly designated by the term "drilling mud," is essentially liquid.

The surface installation 17 comprises a device 41 for supporting and driving the rotation of the drilling tool 15, a injector 43 for injecting drilling liquid and a vibrating screen 45.

The injector 43 is hydraulically connected to the injection head 31 to introduce the drilling fluid and cause it to circulate in the inner space 35 of the drill string 29.

The inner space 35 emerges opposite the drill head 27 so that the drilling fluid lubricates the bit 33 and goes up in the cavity 14 along the duct 13 up to the well head 23, evacuating the solid drilling debris collected.

The drilling fluid present in the cavity 14 maintains a hydrostatic pressure in the cavity, which prevents the walls defining the cavity 14 not covered by the duct 13 from breaking and which also prevents the eruptive release of hydrocarbons in the cavity 14.

The circulation duct 25 or flowline is hydraulically connected to the cavity 14 through the well head 23 to collect the drilling fluid coming from the cavity 14. It is for example formed by an open neck or by a closed tubular duct.

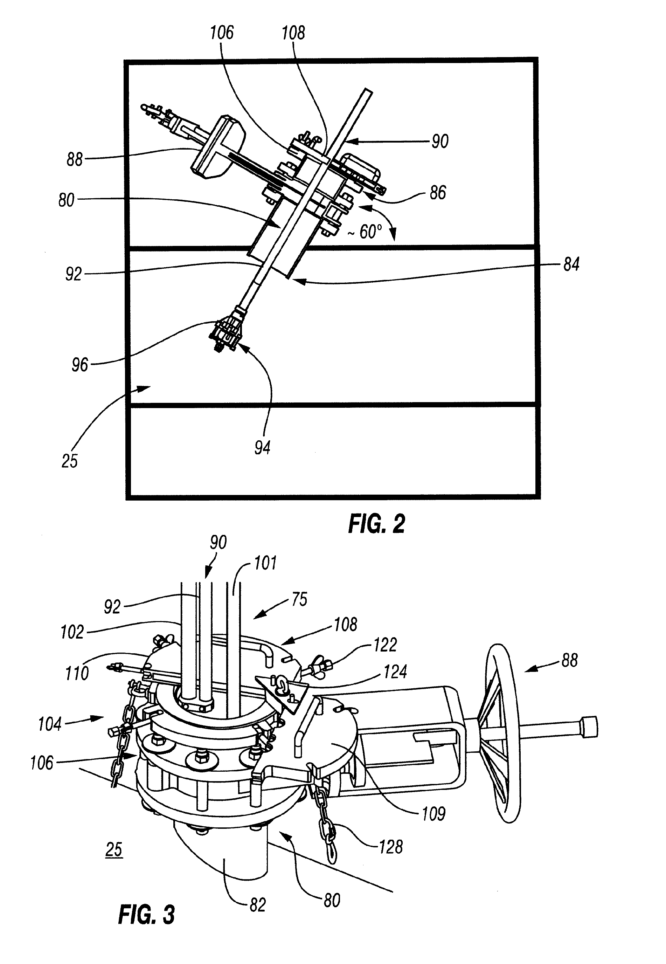

In the example illustrated in Figure 2, the duct 25 is a closed tubular duct. It comprises a main segment for circulation of the fluid and an opening emerging transversally in the main segment to allow the sampling of the fluid. Alternatively, the duct may have a closed section portion situated at the output of the drilling duct, this closed-section portion being followed by an open section portion. Any other configuration of the duct 25 including a closed section portion may be used in relation with the disclosure.

The vibrating screen 45 collects the fluid charged with drilling residue that comes out of the circulation duct 25 and separates the liquid from the solid drilling residue.

The analysis system 19 comprises a device 71 for extracting the gases contained in the drilling fluid, and a device 73 for transporting and analyzing gases extracted from the drilling fluid in the extraction device 71.

The extraction device 71 is arranged in the vicinity of the wellhead 23, in an explosive zone. A sampling system 75 for continuously sampling drilling fluid circulating in the duct 25 guides the sampled drilling fluid to an enclosure 77 for extracting gases outside the fluid sampled by the sampling system 75, via a conveyor 79 for conveying the sampled fluid between the sampling system 75 and the extraction enclosure 77. The sampling system will be described in further details in view of the other figures.

The conveyor 79 comprises a connecting tubing 159 and a pump 161.

The tubing 159 connects the sampling system 75 to the inlet of the pump 161. It emerges upstream in the sampling system 75.

The pump 161 is for example a peristaltic pump, able to convey the sampled drilling fluid towards the enclosure 77, at an adjustable flow rate.

In the example illustrated in Figure 1, the enclosure 77 comprises a container 163, a duct 165 for bringing mud into the container 163, a duct 167 for discharging mud outside the container 163, an inlet 169 for introducing a gas into the container 163, and an outlet 171 for extracting extracted gases.

The container 163 has an inner volume for example between 0.4 liters and 3 liters. This container 163 comprises a lower portion 173 in which the drilling fluid coming from the sampling system 75 circulates and an upper portion 175 that has a vapor space.

In this example, the container 163 is provided with a rotary mixing unit 177 that protrudes in the inner volume to be submerged in and agitates the fluid when it rotates.

The mud intake duct 165 extends between the outlet of the pump 161 and the lower portion 173 of the container 163. This intake duct 165 can be provided with a heater for heating the fluid (not shown), in order to bring the temperature of the fluid to values between 25°C and 120°C, preferably between 60°C and 90°C.

The evacuation duct 167 extends between an overflow passage 187, formed in the upper portion 175 of the container 163, and a retention tank 189 designed to receive the fluid evacuated outside the extraction device 71.

The evacuation duct 167 is advantageously bent to form a siphon 193 emerging opposite the tank 189 above the level of the liquid contained in said tank.

The fluid introduced into the container 163 via the intake duct 165 is evacuated by overflow into the evacuation duct 167 through the overflow passage 187. Part of the evacuated fluid temporarily resides in the siphon 193 of the evacuation duct 167, which prevents gas from entering the upper portion 175 of the container 163 via the evacuation duct 167.

Gas is therefore introduced into the container 163 solely via the inlet 169 for introducing the gas.

The fluid collected in the retention tank 189 is recycled towards the injector 43 by a recirculation duct 198.

In the example illustrated by FIG. 1, the gas introduced by the inlet 169 is formed by the air around the installation, for instance at atmospheric pressure. Alternatively, this gas is another gas such as nitrogen or helium.

The transport and analysis device 73 comprises a line 201 for transporting extracted gases and an analyzer 203 for qualifying and quantifying the gases extracted from the fluid.

The transport line 201 connects the container 163, arranged in the vicinity of the wellhead 23, in an explosive zone, to the analyzer 203 arranged, away from the wellhead 23, in a non-explosive zone, for example a pressurized cabin.

The transport line 201 is advantageously made with a base of a metal or polymer material, in particular polyethylene and/or polytetrafluoroethylene (PTFE). The line 201 for example has a length varying between 10 meters and 500 meters.

The analyzer 203 comprises a suction device (not shown) for conveying the gases extracted outside the enclosure 77 through the transport line 201 and an instrumentation (not shown) that enables the detection and quantification of one or several extracted gases coming from the transport line 201.

This instrumentation for example comprises infrared detection devices for quantifying carbon dioxide, chromatographs (flame ionization detectors, FID) for detecting hydrocarbons, or TCD (Thermal Conductivity Detectors) depending on the gases to be analyzed.

It may also comprise a chromatography and gas system coupled to a mass spectrograph, this system being designated by the abbreviation "GC-MS" or any type of analyzer such as optical or photo-acoustic analyzer. It may also comprise an isotopic analysis device, as described in application EP 1 887 343 by the Applicant.

The simultaneous detection and quantification of a plurality of gases is therefore possible.

The sampling system 75 will now be described in more details. It comprises a first portion 80 connected in the flowline 25 and in fluid communication with the flowline 25, for instance via an opening in the wall of the duct 25. The first portion 80 comprises for instance an auxiliary duct 82 welded to the flowline 25 at a proximal end and having a first opening, opening in the flowline 25 at the proximal end 84 and a second opening at the opposite end 86, referred to as distal end, opening on the outside of the duct. The axis of the auxiliary duct may be tilted relative to the axis of the flowline, for instance by a angle between 30 and 80°, more particularly about 60°. Preferably, the auxiliary duct 82 is tilted so that the distal end is situated downstream relative to the proximal end.

The first portion 80 further comprises a closing device 88 movable between an open position in which the first portion defines a fluid passage between the outside and the closed-section portion of the flowline and a closed position in which the closing device closes the fluid passage. The closing device 88 may be a valve fixed at the distal end of the auxiliary duct and being configured to close the second opening if needed.

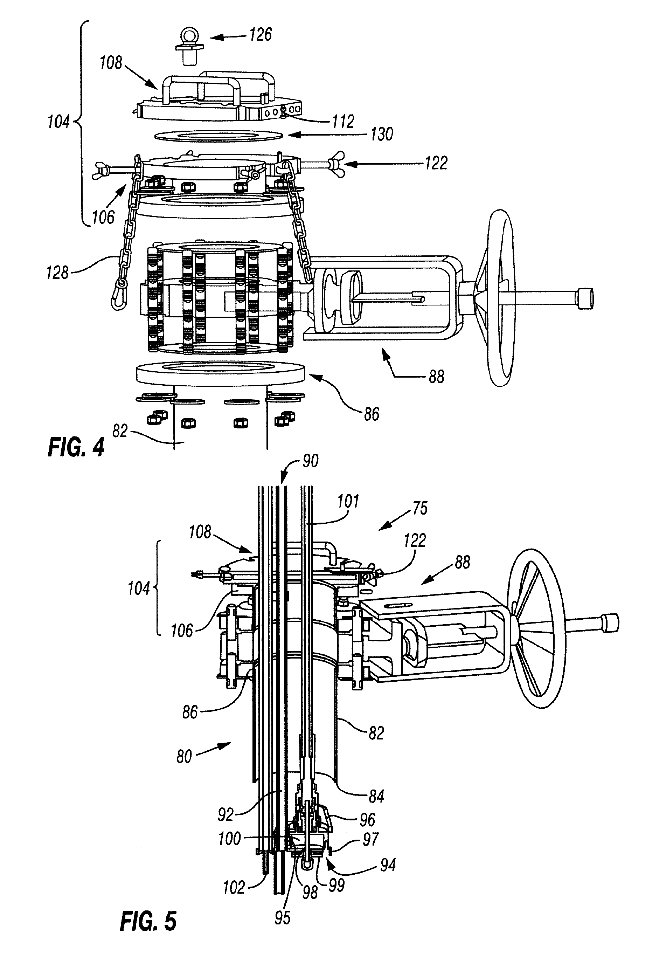

The sampling system 75 further comprises a second portion 90, configured to have a proximal end inserted in the first portion when the closing device is in open position. The second portion comprises at least a transport pipe 92 for transporting sampled drilling fluid to the extraction chamber. This transport pipe is connected to the pump 161 (directly or with the interposition of other lines) at a distal end. It may be connected or constitute part of the tubing 159. At the proximal end of the transport pipe 92, is positioned a collection chamber 94 having a essentially cylindrical shape, as shown on FIG. 5. The collection chamber 94 is connected to the transport pipe via an opening 95 situated in a lateral wall of the collection chamber opening in the transport pipe 92. The transport pipe 92 is plugged at its proximal end so that the sampled drilling fluid does not fall back by effect of gravity in the flowline 25 and the sampled drilling fluid is then transported to the extractor 71 as explained before.

The collection chamber 94 comprises a filtration member 98 also called strainer for avoiding that cuttings enter the collection chamber 94 situated in its lower wall. The filtration member 98 comprises openings that do not exceed a threshold diameter. The collection chamber may also comprise a blade 99 such as a rotating blade, shown on FIG.5, situated below the filtration member 98 to remove mud stuck on the lower wall of the collection chamber 94 and avoid to plug the filtration member 98. The blade 99 is rotated via a drive shaft 101, for instance a flexible drive shaft that goes through the collection chamber.The architecture of the collection chamber and the blade 99 are similar to the one disclosed in patent US 5,090,256 hereby incorporated by reference.

The lateral wall of the collection chamber 94 may further be reinforced by a shield 97 protruding towards the lower end of the collection chamber further than the lower wall of the chamber to protect the chamber from being damaged by cuttings. The second portion may also include a guiding cone 96 for guiding the second portion, out of the first portion 80. The guiding cone 96 is situated at the upper end of the collection chamber 94 with its biggest diameter closer to the collection chamber.

The second portion may also comprise a probe 102 for measuring parameters relative to the drilling fluid in situ, such as level of the drilling fluid, etc.

The second portion 90 may also comprise a protection member 100 protruding relative to the collection chamber 94 in the direction of the axis of the transport pipe 92, situated at the proximal end of the transport pipe. This protection member 100 is the first to be in contact with the lower end of the flowline when inserted in the flowline. Damages to the collection chamber and the probe 102 at insertion in the pipe are then avoided.

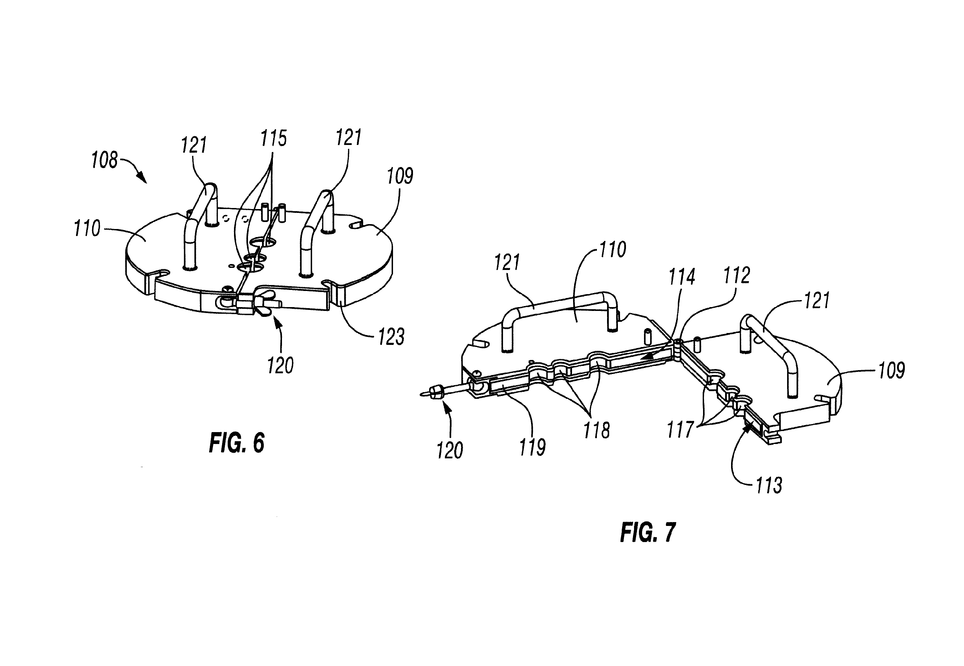

The sampling system 75 also includes a retaining device 104. The retaining device comprises a retaining collar 106 that may be releasably mounted on the closing device 88. It also comprises an attaching part 108. The attaching part comprises two members 109, 110 rotatably mounted to each other via an hinge 112 and respective contacting surface 113, 114. The members 109,110 are movable relative to each other between a first position, represented on FIG. 6, in which respective contacting surfaces of each member are in contact and define at least an opening 115 for receiving the second portion and a second position, shown on FIG. 7, in which the contacting surfaces are at least partly separated from each other. In this example, the attaching part comprises 3 openings receiving respectively the transport pipe 92, the driving shaft 101 and the probe 102.

Each member comprise recesses 117, 118 that define the opening when the members are in a first position. Recesses 117, 118 are configured to face each other when the attaching part is in the first position. The contacting surfaces may be coated by a seal 119 that extends continuously on the contacting surface and on the seal, for instance made of PTFE. It also comprises a fixing member 120 for fixing the members in the first position. The fixing member 120 may be configured so as not to be detachable from the attaching part 108 which may facilitate the mounting of the retaining device 104. A handle 121 is also provided on each member to facilitate manutention of the attaching part.

The attaching part 108 may be releasingly mounted on the retaining collar 106 via second fixing members 122 such as screws, engaging in complementary openings 123 of the attaching part 108. The second fixing members 122 may be configured so as not to be detachable from the retaining collar 106 which may facilitate the mounting of the retaining device 104.

The retaining device may also comprise additional elements such as a locking device 124 for maintaining the members 109, 110 in the second position. The locking device may be a wedge that is inserted between the contacting surfaces of the members 109, 110 when the attaching part is in the second position to prevent them from contacting.

It may also comprise a plug 126 for plugging one of the openings 115 defined by both members 109, 110 in first position. This plug may be made of a sealing material and may seal an opening that is not used to receive an element of the second portion or more simply of a metallic part having a shape cooperating with the opening. In this case, the sealing is performed via seals 119 situated on the contactings surfaces113, 114. By using this plug, the same retaining device may be a standard device that enables to retain several types of second portions, for instance without protection member or probe.

The additional elements 124, 126 may also be provided on the retaining collar, so that they are not detachable from the retaining collar, for instance via chains 128 or ropes.

Now the operation of the sampling device and the installation mentioned above will be described in more details.

First, before installing the second portion of the sampling device in the flowline, when the extraction and analysis of gas in the drilling fluid is not needed, the closing device is in closed position so that no fluid escapes from the flowline via the first portion. The retaining collar 106 is already mounted on the closing device 88.

To launch the analysis of the gas, the closing device 88 is moved in the open position and the attaching part 108 is positioned on the retaining collar 106. The members 109, 110 are positioned in the second position and the wedge 124 is inserted between the contacting surfaces to keep them apart from each other. The second portion, namely in this example the transport pipe 92, the protection member 100 and the probe 102, is then inserted in the recesses 118 of one of the member 110, as shown on FIG. 3.

The second portion is more particularly lowered into the first portion 82 until the protection member 100 reaches the bottom wall of the flowline. The second portion 90 is then risen again of a few centimeters so that the collection chamber is easily set in the drilling mud circulating in the flowline (in the half bottom of the flowline, for instance) without damaging the collection chamber during the setting.

When the position of the collection chamber 94 in the flowline is appropriate, the wedge 124 is removed and the members 109, 110 are moved in the first position, so that the contacting surfaces 113, 114 of each of the members 109, 110 are in contact. The fixing member 120 is fixed and the members 109, 110 are locked in the first position. In this position, the second portion 90 is locked in the attaching part 108 and sealed relative to the attaching part, in particular thanks to the seal 119.

The attaching part 108 is then fixed to the retaining collar by aligning the openings 123 with the screws 122 and by locking the attaching part 108 on the retaining collar 106. Once the attaching part 108 fixed to the retaining collar 106, which may take place with interposition of a sealing ring 130 as shown on FIG 4, the retaining collar 106 and attaching part 108 and second portion 90 block the fluid passage defined by the first portion even though the closing device of the first portion is in open position. The fluid may escape from the flowline via the second portion and more particularly the transport line 92

When the second portion is fixed relative to the first portion, with one of its end in the flowline, its free end is connected to the pump 161 and the suction and sampling of the drilling fluid can begin.

When the sampling is performed, drilling fluid is extracted from the flowline and is sucked in the extraction chamber. The gas included in the drilling fluid is then extracted from the drilling fluid in any convenient way, for instance as disclosed in application US 7,032,444.

Once extracted, the gas is transported to the analysis system that analyzes the content of several gas such as hydrocarbons, carbon dioxide, etc., contained in the drilling fluids from the extracted gas. Any type of analysis system may be part of the installation (spectroscopy, photo-acoustic measurements, mass chromatography, etc.), as already disclosed hereinabove.

During the sampling of the fluid, several parameters may be monitored, such as the level in the flowline 25. When the parameter is above or below a predetermined threshold, for instance when the level in the flowline 25 is above a predetermined level, the sampling is stopped and the closing device is set in the closing position. Other parameters such as pressure in the transport pipe may also be monitored and constitute a triggering event for the stopping of the sampling.

In this case or when it is decided to stop the sampling, for instance for maintenance, the fixing members 122 are released and the attaching part 108 is separated from the retaining collar 106. The second portion 90 and the retaining members are then removed together from the first portion 82. The closing device 88 is then moved to the closed position.

Although the preceding description has been described herein with reference to particular means, materials and embodiments, it is not intended to be limited to the particulars disclosed herein; rather, it extends to all functionally equivalent structures, methods and uses, such as are within the scope of the appended claims.

For instance, the configuration of the retaining device may be different from what is disclosed here. The retaining device may not be releasably mounted on the first portion. The configuration of any of the first or second portion or of the retaining device is not limited to what has been disclosed. The installation that has been described is also only an embodiment among others that is not limiting regarding the scope of the claims. Further, the insertion and removing of the second portion in the first portion may not be performed as disclosed.

The disclosure at least relates to a system for sampling a drilling fluid exiting the wellbore in a portion of a flowline having a substantially closed section, wherein the system comprises :
- a first portion connected in the closed-section portion of the flowline, comprising a closing device movable between an open position in which the first portion defines a fluid passage between the outside and the closed-section portion of the flowline and a closed position in which the device closes the fluid passage,
- a second portion configured to be inserted in the first portion, with the closing device in open position, defining a fluid passage between the outside and the closed-section portion of the flowline;
- a retaining device for fixing the second portion relative to the first portion ,
in which the second portion and the retaining device are configured so as to close the fluid passage defined by the first portion, when the second portion is positioned in the first portion.

The retaining device may be configured so as to releasably fix the second portion relative to the first portion.

At least part of the retaining device may be releasably mounted on the first portion.

The retaining device may include at least two members movable relative to each other between a first position in which respective contacting surfaces of each member are in contact and define at least an opening for receiving the second portion and a second position in which the contacting surfaces are at least partly separated from each other. A recess may be provided in the contacting surface of at least one of the members, the recess defining at least partly the opening. At least one of the contacting surface may be provided with a seal.

The members may be rotatably connected to each other, for instance via an hinge. The retaining device may also comprise at least a first fixing element for fixing the members in the first position and/or at least a second fixing element for releasably connecting at least one member to the first portion, directly or with interposition of another part.

The sampling system may comprise a locking element for locking the members in the second position, ie for preventing from reaching the first position.The locking element may be a wedge.

The members may define at least two openings. The retaining device may further comprise a plug for plugging at least one of the openings.

At least one of the locking elements or fixing members or plug is attached to one of the retaining device, first portion and second portion so that the system may be easily manipulated. They may be fixed via ropes or chains.

The second portion may comprise at least a transport pipe for guiding the sampled drilling fluid outside of the closed section flowline, the transport pipe being configured to be inserted inside of the closed section flowline.

The second portion comprise a collection chamber at the end of the transport pipe for collecting the drilling fluid. The collection chamber may comprise a filtration member to filter the drilling fluid entering the collecting chamber and may be reinforced for protection of the filtration member.

The second portion may also comprise a protection member protruding relative to the collection chamber in a direction corresponding to the axial direction of the transport pipe.

The second portion may also comprise a probe, such as a level sensor.

Further, the axis of the first and/or second portions may be tilted relative to the axis of the flowline.

Installation for analyzing gas extracted from a drilling fluid exiting a wellbore comprising
- a flowline for removing drilling fluid from the wellbore, the flowline having at least a closed section portion,
- a system for sampling the drilling fluid in the closed section portion of the flowline according to the disclosure,
- a system for extracting at least a gas from the sampled drilling fluid, connected to the sampling system,
- a system for analyzing the gas extracted from the drilling fluid, connected to the extraction system.

These systems are disclosed upstream to downstream.

The disclosure also relates to a method for sampling a drilling fluid exiting a wellbore in a closed section flowline with a system according to the disclosure, comprising:
- opening the closing device,
- inserting the second portion of the sampling system in the first portion until at least part of the second portion is situated in the closed section flowline,
- fixing the second portion relative to the first portion,
- connecting the second portion to a suction device to extract drilling fluid from the closed section flowline.

The method may also comprise :
- if a predetermined configuration of the wellbore is detected, disconnecting the second portion from the suction device,
- detaching the second portion from the first portion and removing the second portion from the first portion,
- closing the closing device.

The predetermined configuration may be pressure or level in the closed section flowline is above a predetermined threshold

## Claims

1. System for sampling a drilling fluid exiting the wellbore in a portion of a flowline having a substantially closed section, wherein the system comprises :
- a first portion connected in the closed-section portion of the flowline, comprising a closing device movable between an open position in which the first portion defines a fluid passage between the outside and the closed-section portion of the flowline and a closed position in which the device closes the fluid passage,
- a second portion configured to be inserted in the first portion, with the closing device in open position, defining a fluid passage between the outside and the closed-section portion of the flowline;
- a retaining device for fixing the second portion relative to the first portion,
wherein the second portion and the retaining device are configured so as to close the fluid passage defined by the first portion.

2. Sampling system according to claim 1, wherein the retaining device is configured so as to releasably fix the second portion relative to the first portion.

3. Sampling system according to any preceding claims, wherein at least part of the retaining device is releasably mounted on the first portion.

4. Sampling system according to the preceding claims, wherein the retaining device comprises at least two members movable relative to each other between a first position in which respective contacting surfaces of each member are in contact and define at least an opening for receiving the second portion and a second position in which the contacting surfaces are at least partly separated from each other.

5. Sampling system according to the preceding claim, wherein a recess is provided in the contacting surface of at least one of the members, the recess defining at least partly the opening.

6. Sampling system according to the preceding claim, wherein the members are rotatably connected to each other.

7. Sampling system according to any of claims 4 to 6, wherein at least one of the contacting surface is provided with a seal.

8. Sampling system according to any of claims 4 to 7, wherein the members define at least two openings, the retaining device further comprising a plug for plugging at least one of the openings.

9. Sampling system according to any of the preceding claims, wherein the second portion comprises at least a transport pipe for guiding the sampled drilling fluid outside of the closed section flowline, the transport pipe being configured to be inserted inside of the closed section flowline.

10. Sampling system according to the preceding claim, wherein the second portion comprises a collection chamber at the end of the transport pipe for collecting the drilling fluid.

11. Sampling system according to the preceding claim, wherein the second portion comprises a protection member protruding relative to the collection chamber in a direction corresponding to the axial direction of the transport pipe.

12. Installation for analyzing gas extracted from a drilling fluid exiting a wellbore comprising :
- a flowline for removing drilling fluid from the wellbore, the flowline having at least a closed section portion,
- a system for sampling the drilling fluid in the closed section portion of the flowline according to any preceding claim,
- a system for extracting at least a gas from the sampled drilling fluid, connected to the sampling system,
- a system for analyzing the gas extracted from the drilling fluid, connected to the extraction system.

13. Method for sampling a drilling fluid exiting a wellbore in a closed section flowline with a system according to any preceding claim, comprising:
- opening the closing device,
- inserting the second portion of the sampling system in the first portion until at least part of the second portion is situated in the closed section flowline,
- fixing the second portion relative to the first portion,
- connecting the second portion to a suction device to extract drilling fluid from the closed section flowline.

14. Method according to the preceding claim comprising:
- if a predetermined configuration of the wellbore is detected, disconnecting the second portion from the suction device,
- detaching the second portion from the first portion and removing the second portion from the first portion,
- closing the closing device.

15. Method according to the preceding claim, wherein the predetermined configuration is pressure or level in the closed section flowline is above a predetermined threshold.
